# EUROPEAN PATENT SPECIFICATION

(11) **EP 4 245 331 B1**
(45) Date of publication and mention of the grant of the patent: **06.05.2026**
(21) Application number: 23161710.1
(22) Date of filing: 14.03.2023
(51) Int. Cl.: A61M 1/36, B04B 13/00, B04B 5/04

(54) **CENTRIFUGE WITH CONTACTLESS POWER SOURCE FOR ELECTRONIC ACTIVE DEVICE**
ZENTRIFUGE MIT KONTAKTLOSER ENERGIEQUELLE FÜR EINE ELEKTRONISCHE AKTIVE VORRICHTUNG
CENTRIFUGEUSE AVEC SOURCE D'ALIMENTATION SANS CONTACT POUR DISPOSITIF ÉLECTRONIQUE ACTIF

(30) Priority: 17.03.2022 US 202263320898 P
(43) Date of publication of application: 20.09.2023
(73) Proprietor: Fenwal, Inc., Lake Zurich, IL 60047 (US)
(72) Inventor: WEBER, Mark C., Lake Zurich, 60047 (US); ERN, Mark, Lake Zurich, 60047 (US); KAST, Michael J., Lake Zurich, 60047 (US); HAN, James C., Lake Zurich, 60047 (US)
(74) Representative: Maikowski & Ninnemann Patentanwälte Partnerschaft mbB

(56) References cited:
- WO-A1-03/000380
- WO-A1-2008/105972
- WO-A1-2021/148831
- WO-A2-2004/018021
- KINAHAN DAVID J ET AL: "Wireless closed-loop control of centrifugo-pneumatic valving towards large-scale microfluidic process integration", 2018 IEEE MICRO ELECTRO MECHANICAL SYSTEMS (MEMS), IEEE, 21 January 2018 (2018-01-21), pages 1213 - 1216, XP033335853, DOI: 10.1109/MEMSYS.2018.8346781

## Description

### TECHNICAL FIELD

The invention relates to centrifugal systems and apparatus that includes a contactless power source for powering active devices on the rotating component of the centrifuge, and more particularly, for powering a sensor on a rotating component wherein the sensor has wireless data transfer capabilities.

### BACKGROUND

Today people routinely process whole blood and blood components by centrifugation into its various therapeutic components, such as red blood cells, platelets, and plasma. For example, WO 03/000380 discloses a blood separation apparatus that includes multiple cassettes on a shaft for the simultaneous separation of multiple blood units.

Conventional blood processing centrifuges use electrically conductive slip rings to provide electrical power to active devices on the rotating components of the centrifuge. Such slip rings also are used to transmit data between the active device and another device that is not located on the rotating component, such as a controller. For example, WO 2004/018021 discloses an apparatus for blood component separation, including a rotor rotated by a motor. The apparatus also includes a slip ring to supply power to elements on the rotating rotor. However, the mechanical nature of slip rings can be unreliable and limits information that can be transferred.
WO 2021/148831 discloses a centrifuge rotor for centrifuging a sample. The centrifuge rotor includes a motor below it and a contactless power transmitter above it. KINAHAN DAVID J ET AL: "Wireless closed-loop control of centrifugo-pneumatic valving towards large-scale microfluidic process integration", 2018 IEEE MICRO ELECTRO MECHANICAL SYSTEMS (MEMS), IEEE, 21 January 2018 (2018-01-21), pages 1213-1216, XP033335853, DOI: 10.1109/MEMSYS.2018.8346781 discloses an electronically controlled "Lab-on-a-Disc" system with a rotating disc platform. The system includes a motor below the rotating disc platform and a wireless data and power transmitter above the rotating disc platform.

There remains a need for reliable energy to and data exchange (send and/or receive) with active devices located on the rotating component of a centrifuge.

### SUMMARY

There are several aspects of the present subject matter which may be embodied separately or together in the devices, systems, and methods described and/or claimed below. These aspects may be employed alone or in combination with other aspects of the subject matter described herein. The scope of the invention is as defined in the appended claims. For purposes of this description and claims, unless otherwise expressly indicated, "blood" is intended to include whole blood and blood components, such as concentrated red cells, plasma, platelets, and white cells, whether with or without anticoagulant or additives.

The following summary is to acquaint the reader generally with various potential aspects of the present subject matter, and is non-limiting and non-exclusive with respect to the various possible aspects or combinations of aspects. Additional aspects and features may be found in the detailed description herein and/or in the accompanying Figures.

The invention provides a centrifuge assembly for processing blood and/or components according to appended claim 1. The centrifuge assembly includes a base and a rotating component that rotates relative to the base. The assembly includes a contactless, such as a wireless, power source including a power source transmitter associated with the base and a power source receiver associated with the rotating component. The power source transmitter wirelessly transmits power to the power source receiver. An active electronic device is associated with the rotating component and operatively connected to the power source receiver. The power source receiver provides power to the active electronic device.

The invention also provides a non-therapeutic, non-surgic method of processing blood and/or components with a centrifuge according to appended claim 13. The method includes flowing blood and/or blood components to a rotating component of a centrifuge assembly and rotating the rotating component of the centrifuge assembly relative to a base. The method further includes powering an active electronic device associated with the rotating component using a contactless power source.

These and other aspects of the present subject matter are set forth in the following detailed description of the accompanying drawings.

### BRIEF DESCRIPTION OF THE DRAWINGS

Fig. 1 is a side elevation view of an exemplary processing system that includes a centrifuge assembly in accordance with the present disclosure;
Fig. 2 is a front perspective view of the processing system shown in Fig. 1;
Fig. 3 is an exploded perspective view of the components of the centrifuge assembly;
Fig. 4 is an enlarged perspective view of the rotating components of the centrifuge assembly shown in its suspended operating position;
Fig. 5 is a side sectional view of the rotating components of the centrifuge assembly taken generally along line 5-5 in Fig. 4; and
Fig. 6 is an example of an active device on the rotating component of the centrifuge assembly.

### DETAILED DESCRIPTION OF THE ILLUSTRATED EMBODIMENTS

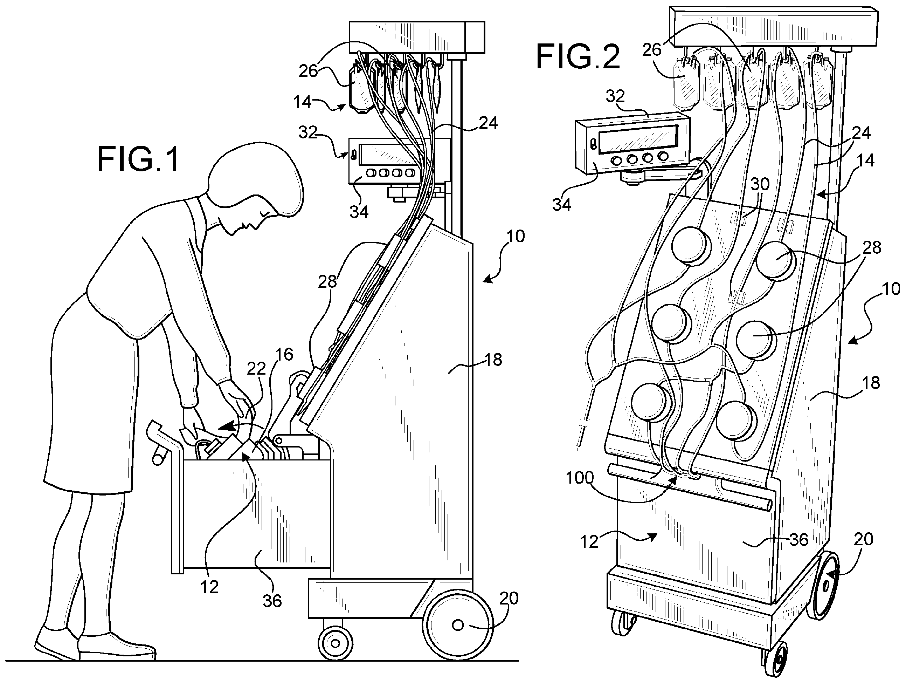

Figs. 1 and 2 show an exemplary centrifugal processing system 10 that employs a centrifuge assembly 12 of the present disclosure. The system 10 can be used for processing various fluids. The system 10 is particularly well suited for processing whole blood and other suspensions of cellular materials that are subject to trauma.

The system 10 includes a centrifuge assembly 12 and an associated fluid processing assembly 14. The centrifuge assembly 12 is a durable equipment item. The fluid processing assembly 14 is a single use, disposable item that the user loads on the centrifuge assembly 12 before beginning a processing procedure (as Fig. 1 generally shows) and removes from the centrifuge assembly 12 upon the completing the procedure.

The centrifuge assembly 12 comprises a centrifuge 16 mounted for rotation within a cabinet 18. The user maneuvers and transports the cabinet 18 upon the associated wheels 20. It should be appreciated that, due to its compact form, the centrifuge assembly 12 also could be made as a tabletop unit.

As Figs. 1 and 2 show, the cabinet 18 includes a sliding drawer 36 that holds the centrifuge 16. As Fig. 1 shows, the user opens the drawer 36 to access the centrifuge 16 for inserting and removing the processing chamber 22. As Fig. 2 shows, the user closes the drawer 36 when conducting a processing operation. The processing assembly 14 comprises a processing chamber 22 mounted on the centrifuge 16 for rotation (as Fig. 1 shows). An associated fluid circuit 24 conveys fluids to and from the processing chamber 22. The fluid circuit 24 has several fluid containers 26. As Fig. 2 shows, in use, the containers 26 hang from a support pole outside the cabinet 18. The fluid circuit 24 transits several peristaltic pumps 28 and clamps 30 on the face of the cabinet 18. The fluid circuit 24 enters an access opening 100 leading to the processing chamber 22 mounted within the cabinet 18. In the illustrated environment, the fluid circuit 24 preconnects the processing chamber 22 with the containers 26, forming an integral, sterile unit closed to communication with the atmosphere.

The centrifuge assembly 12 includes a processing controller 32 that coordinates the operation of the centrifuge 16. The processing controller 32 preferably uses an input/output terminal 34 to receive and display information relating to the processing procedure.

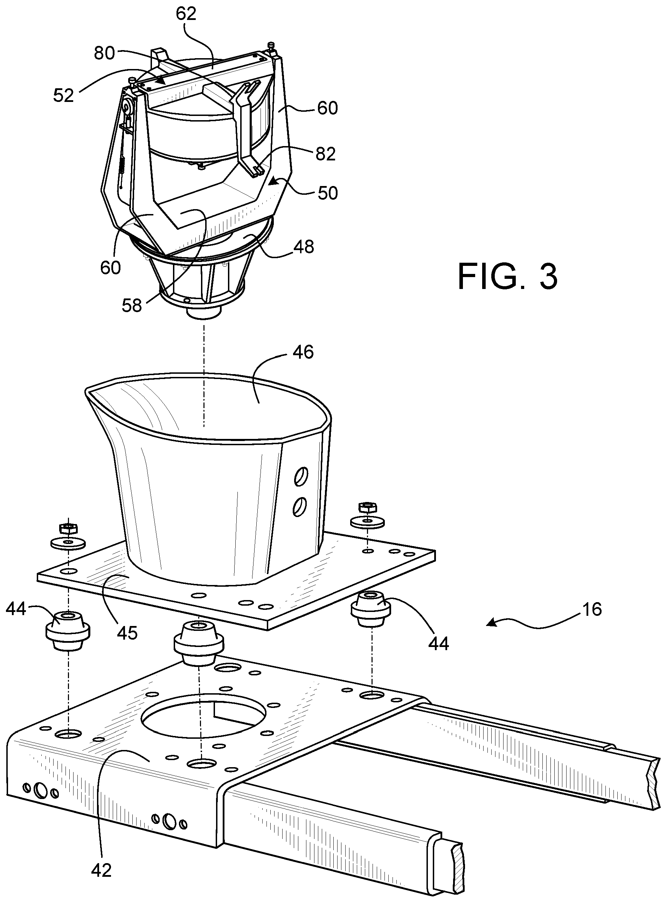

As Fig. 3 shows, the centrifuge 16 includes a base 42 that supports a plate 45 mounted upon flexible isolation mounts 44. The flexible mounts 44 structurally isolate the components mounted on the plate 45 from the rest of the centrifuge 16, by dampening vibration and oscillation caused by these plate-mounted components. The components mounted on the plate 45 make up the isolated mass of the centrifuge 16.

A nonrotating outer housing or bucket 46 is mounted on the plate 45. The bucket 46 encloses a stationary platform 48, which in turn supports the rotating components of the centrifuge 16.

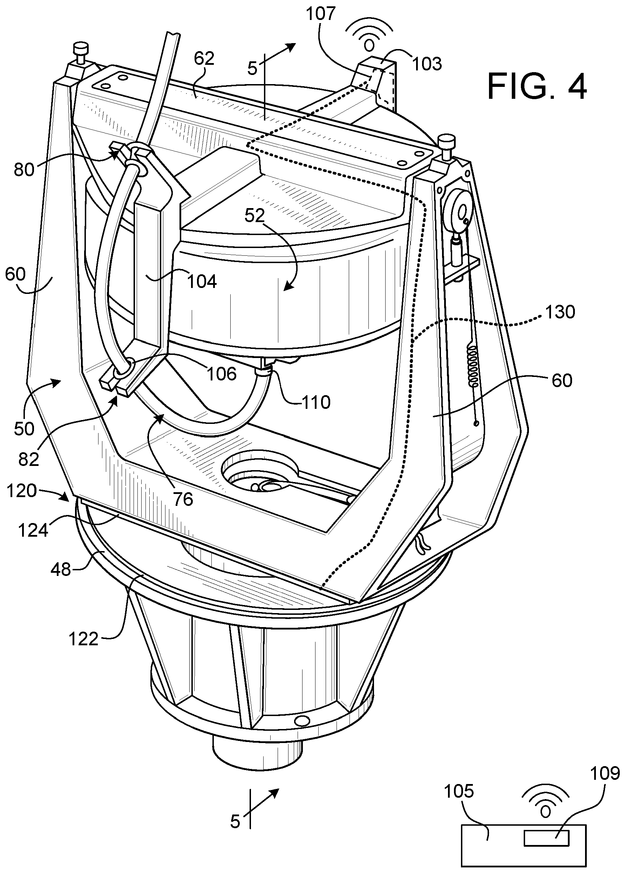

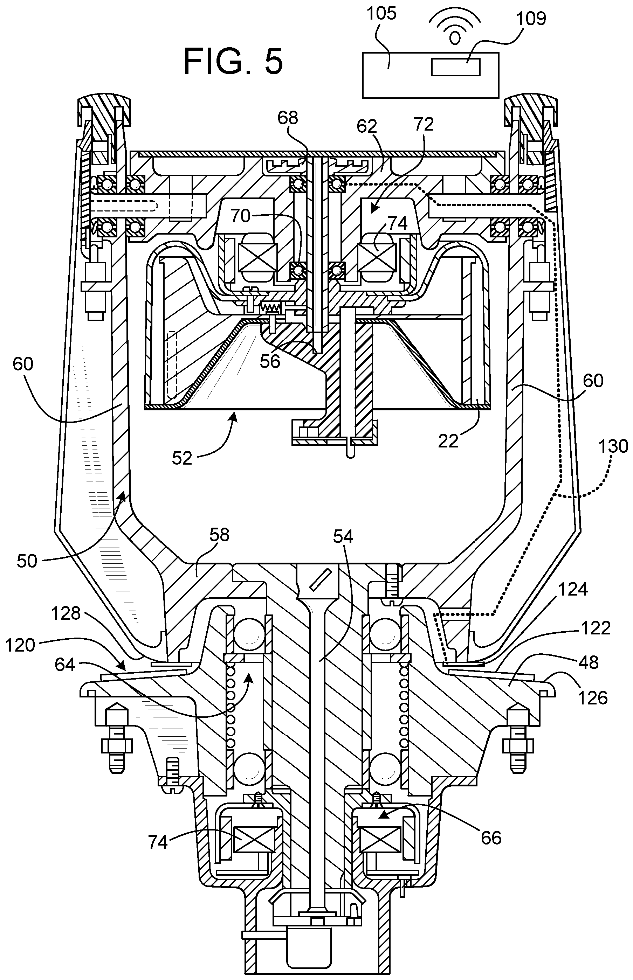

As Figs. 4 and 5 show in greater detail, the rotating components include a centrifuge yoke assembly 50 and a centrifuge chamber assembly 52. The yoke assembly 50 rotates upon the platform 48 on a first drive shaft 54. The chamber assembly 52 rotates on the yoke assembly 50 on a second drive shaft 56. The rotating chamber assembly 52 carries the processing chamber 22.

The yoke assembly 50 includes a yoke base 58, a pair of upstanding yoke arms 60, and a yoke cross member 62 mounted between the arms 60. The base 58 is attached to the first drive shaft 54, which spins on a bearing element 64 about the stationary platform 48. A first electric drive 66 rotates the yoke assembly 50 on the first drive shaft 54.

The chamber assembly 52 is attached to the second drive shaft 56, which spins on a bearing element 68 in the yoke cross member 62. The second drive shaft 56 and the bearing element 68 spin as a unit on ball bearings 70. A second electric drive 72 rotates the centrifuge chamber assembly 52 on the second drive shaft.

The first electric drive 66 and the second electric drive 72 each comprises a permanent magnet, brushless DC motor. As Fig. 5 shows, the stationary platform holds the field coils 74 of the first motor 66, while the yoke base 58 comprises the armature or rotor of the first motor 66. The yoke cross member 62 holds the field coils 74 of the second motor 72, while the chamber assembly 52 comprises the associated armature or rotor.

In the illustrated embodiment, the first electric motor 66 spins the yoke assembly 50 at a predetermined speed of rotation. The second electric motor 72 spins the chamber assembly 52 at the same speed of rotation as the first electric motor 66 in the same direction and about the same axis as the spinning yoke assembly 50.

The yoke assembly 50 includes tube holders 80 and 82 that are carried by yoke cross member 62. An active electronic device 103 also is associated with the yoke 50. In the illustrated embodiment, the yoke cross member 62 carries active electronic device 103, such as a sensor that senses or measures a characteristic of the fluid being processed and/or the centrifuge. In one alternative, the active electronic device 103 may be a blood component detector, which sensors or detects various blood components and/or their interfaces. In another alternative, the active electronic device 103 may be one or more of an interface detector, pressure sensor, temperature sensor, hemolysis sensor, a weight scale or any other suitable electronic device or sensor. In another alternative the active electronic device 103 may include an optical sensor or a camera used to sense or determine characteristics of blood within the centrifuge.

The active electronic device 103 is powered by a contactless power source 120, such as a wireless power source. The contactless power source 120 may include inductive, capacitive, or magneto-dynamic contactless power transfer or any other suitable contactless power transfer. Referring to Figs. 4 and 5, the contactless power source 120 includes a power source transmitter 122 and a power source receiver 124. The power source transmitter 122 is associated with the stationary platform 48 upon which the yoke assembly rotates. In the illustrated embodiment, the power source transmitter 122 is a relatively flat plate which is associated with a surface 126 of the platform 48 facing the yoke 50. For example, the power source transmitter 122 may be located on or embedded in surface 126. The power source receiver 124 is associated with a rotating component of the centrifuge 16, such as the rotating yoke 50. In the illustrated embodiment, the power source receiver 124 is a relatively flat plate that is associated with a surface 128 of the rotating yoke 50 facing the stationary platform 48. For example, the power source receiver 124 may be located on or embedded in surface 128.

The active electronic device 103 is powered by the contactless power source 120. In the illustrated embodiment, the active electronic device 103 is operatively connected to the power source receiver 124 by one or more wires 130. In the embodiment shown, the wire(s) 130 are shown in broken-line to indicate that they are located or run within the structures of the rotating yoke 50. In other embodiments, the wire(s) 130 may run along the outer surfaces of the rotating yoke 50.

During centrifugation, the rotating yoke 50 rotates relative to the stationary platform 48. The power source transmitter 122 on the stationary platform transmits energy to the power source receiver 124. The power source receiver 124 powers the active electronic device 103, which may be sensor. In one embodiment, the active electronic device may be electric drive 72.

The active electronic device 103 may exchange data wirelessly with another device 105 that is not located on the rotating component of the centrifuge. For example, the other device 105 is not located on or associated with yoke 50. The other device 105 may be for example the controller 32 or in the controller 32 (Fig. 1) which is not located on the rotating component. The active electronic device 103 may have a wireless data exchange device 107 that exchanges (sends and/or receives) data to a separate data exchange device 109 of the other device 105. The data may be exchanged via one or more of Bluetooth, Wi-Fi, near field communication or any other suitable wireless peer-to-peer communication. Additionally, the electronic device 103 and the other device 105 also may convert analog data to digital data for wireless transmission.

The combination of the use of the contactless power source 120 and the wireless data exchange results in the ability to use an active electronic device 103 on the rotating component of the centrifuge with little or no mechanical connections. This configuration achieves greater reliability of the active electronic device and less mechanical failures. Furthermore, this combination may be employed in other fields outside of the blood separation. For example, other fields and structures that include an active electronic device on a rotating component.

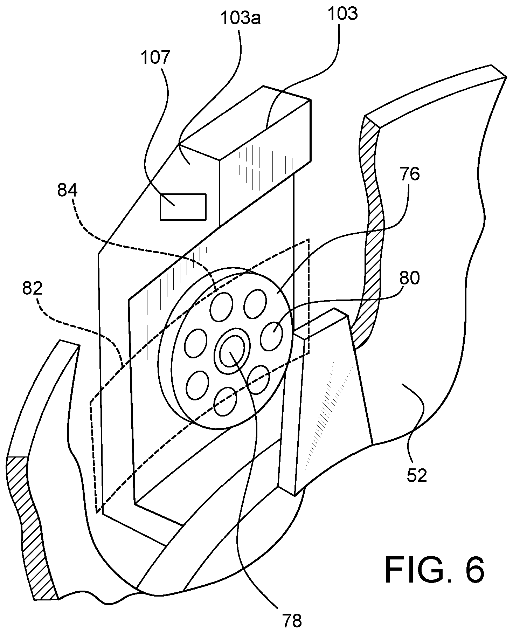

As discussed above, the active electronic device may be any suitable device, such as a sensor or detector that provides information regarding the characteristics of the blood or the operation of the centrifuge. Fig. 6 illustrates an exemplary active electronic device 103. The active electronic device 103 may be an optical sensor 103a that senses the optical characteristics of fluid during the separation process. The sensor 103a may be carried by the cross member 62 (Fig. 4) of the rotating yoke 50. The sensor 103a includes a light source 76, which emits light that is absorbed by the fluid, for example red blood cells. In the illustrated embodiment, the light source 76 includes a circular array of red light emitting diodes 84. Of course, other wavelengths absorbed by RBC, like green or infrared, could be used.

The sensor 103a also includes a light detector 78, which is mounted adjacent to the light source 76. In the illustrated and preferred embodiment, the light detector 78 comprises a PIN diode detector, which is located generally in the geometric center of the circular array of light emitting diodes 84.

The yoke 50 and sensor 103a rotate relative to the platform 48. Furthermore, the centrifuge chamber assembly 52 rotates relative to the sensor 103a. The light source 76 directs light onto the chamber assembly 52. In the illustrated embodiment, the chamber assembly 52 is transparent to the light emitted by the source 76 in the region 86. In the illustrated embodiment, the region 86 comprises a window cut out in the chamber assembly 52. The remainder of the chamber assembly 52 that lies in the path of the sensor 103a comprises a light absorbing material.

The light from the source 76 will thereby pass through the transparent region 86 of the chamber assembly 52 every time the rotating chamber assembly 52 and sensor 103a align. The light is reflected back to toward the sensor, where it is sensed by the detector 78.

As the transparent interface region 86 of the chamber assembly 52 comes into alignment with the sensor 103a, the detector 78 will first sense light reflected through a plasma layer. Eventually, a red blood cell layer will enter the optical path of the sensor 103a. The red blood cell layer absorbs light from the source 76 and thereby reduces the previously sensed intensity of the reflected light. The intensity of the reflected light sensed by the detector 78 represents the amount of light from the source 76 that is not absorbed by the red blood cell layer.

The sensor (active device) may exchange data wirelessly with another device that is not located on the rotating component of the centrifuge. The other device may be for example the controller 32 which is not located on the rotating component. For example, the sensor may have a wireless data exchange member that exchanges (sends and/or receives) data to a separate data exchange member of a device not physically associated with the rotating component of the centrifuge that includes the sensor. Data from the sensor 103a may be wirelessly transmitted to the other device 105. For example, data from sensor 103a may be sent to the controller 32, which then adjusts the centrifuge based on the data. Furthermore, analog data from the sensor 103a may be digitized before being wirelessly transmitted by wireless exchange device 107 to the controller 32.

## Claims

1. A centrifuge assembly for processing blood and blood components, comprising:
a stationary platform (48);
a rotating component located above the stationary platform (48) and rotatable relative to the stationary platform (48), the rotating component comprising a yoke assembly (50) having a yoke base (58);
a drive shaft (54) attached to a yoke base (58);
a motor (66) held by the stationary platform (48), the motor (66) spinning the drive shaft (54) relative to the stationary platform (48), thereby rotating the yoke assembly (50) relative to the stationary platform (48);
a contactless power source (120) including a power source transmitter (122) and a power source receiver (124), wherein the power source transmitter (122) is configured to wirelessly transmit power to the power source receiver (124), the power source transmitter (122) being below the yoke assembly (50) and associated with a surface (126) of the stationary platform (48) facing the yoke assembly (50), and the power source receiver (124) being above the stationary platform (48) and associated with a surface (128) of the yoke assembly (50) facing the stationary platform (48); and
an active electronic device (103) associated with the rotating component and being above the contactless power source (120), the active electronic device (130) being operatively connected to the power source receiver (124), wherein the power source receiver (124) is configured to provide power to the active electronic device (130).

2. The centrifuge assembly of claim 1, wherein the active electronic device (103) is configured to exchange data with another device that is not located on the rotating component.

3. The centrifuge assembly of claim 2, wherein the active electronic device (103) is configured to wirelessly exchange data with the another device (105) not located on the rotating component.

4. The centrifuge assembly of claim 3, wherein the electronic device (103) is configured to wirelessly exchange of data via one or more of Bluetooth, Wi-Fi, and near field communication.

5. The centrifuge assembly of any one of claims 3 and 4, wherein the active electronic device (103) includes a first wireless data exchange device (107) and the another device (105) not located on the rotating component includes a second wireless data exchange device (109), wherein the first and second wireless data exchange devices (107, 109) are configured to exchange data with one another.

6. The centrifuge assembly of any one of claims 2-5, wherein the another device (107) not located on the rotating component comprises a controller.

7. The centrifuge assembly of any one of claims 1-6, wherein the active electronic device (103) comprises a sensor (103a).

8. The centrifuge assembly of claim 7, wherein the sensor (103a) is configured to sense one or more characteristics of blood and/or blood components.

9. The centrifuge assembly of claim 8, wherein the sensor (103a) comprises an optical sensor.

10. The centrifuge assembly of claim 7, wherein the sensor (103a) is configured to sense one or more characteristics of the centrifuge assembly.

11. The centrifuge assembly of any one of claims 1-6, wherein the active electronic device (103) comprises one or more of a blood component detector, an interface detector, pressure sensor, temperature sensor, hemolysis sensor, weight scale and camera.

12. The centrifuge assembly of claim 1, wherein the rotating component further comprises a rotating chamber assembly (52) carrying a processing chamber (22), wherein a fluid circuit (24) is configured to convey fluids to and from the processing chamber (22).

13. A non-surgical and non-therapeutic method of processing blood and/or components with a centrifuge assembly of claim 1, comprising:
flowing blood and/or blood components to the rotating component of the centrifuge assembly;
rotating the rotating component of the centrifuge assembly relative to the stationary platform; and
powering an active electronic device associated with the rotating component with a contactless power source.

14. The non-surgical and non-therapeutic method of claim 13, further including exchanging data between the active electronic device and another device not located on the rotating component.

15. The non-surgical and non-therapeutic method of claim 14, wherein the exchanging of data between the active electronic device and the another device comprises wirelessly exchanging data.

## Patentansprüche

1. Zentrifugenanordnung zur Verarbeitung von Blut und Blutkomponenten, umfassend:
eine stationäre Plattform (48);
eine oberhalb der stationären Plattform (48) angeordnete rotierende Komponente, die relativ zu der stationären Plattform (48) drehbar ist, wobei die rotierende Komponente eine Jochanordnung (50) mit einer Jochbasis (58) umfasst;
eine Antriebswelle (54), die an einer Jochbasis (58) befestigt ist;
einen Motor (66), der von der stationären Plattform (48) gehalten wird, wobei der Motor (66) die Antriebswelle (54) relativ zu der stationären Plattform (48) dreht und dadurch die Jochanordnung (50) relativ zu der stationären Plattform (48) rotiert;
eine kontaktlose Stromquelle (120), die einen Stromquellensender (122) und einen Stromquellenempfänger (124) umfasst, wobei der Stromquellensender (122) dazu eingerichtet ist, drahtlos Strom an den Stromquellenempfänger (124) zu übertragen, wobei der Stromquellensender (122) unterhalb der Jochanordnung (50) angeordnet und einer der Jochanordnung (50) zugewandten Oberfläche (126) der stationären Plattform (48) zugeordnet ist, und wobei der Stromquellenempfänger (124) oberhalb der stationären Plattform (48) angeordnet und einer der stationären Plattform (48) zugewandten Oberfläche (128) der Jochanordnung (50) zugeordnet ist; und
eine aktive elektronische Vorrichtung (103), die der rotierenden Komponente zugeordnet ist und sich oberhalb der kontaktlosen Stromquelle (120) befindet, wobei die aktive elektronische Vorrichtung (130) operativ mit dem Stromquellenempfänger (124) verbunden ist, wobei der Stromquellenempfänger (124) dazu eingerichtet ist, die aktive elektronische Vorrichtung (130) mit Strom zu versorgen.

2. Zentrifugenanordnung nach Anspruch 1, wobei die aktive elektronische Vorrichtung (103) dazu eingerichtet ist, Daten mit einer anderen Vorrichtung auszutauschen, die sich nicht auf der rotierenden Komponente befindet.

3. Zentrifugenanordnung nach Anspruch 2, wobei die aktive elektronische Vorrichtung (103) dazu eingerichtet ist, Daten drahtlos mit der anderen Vorrichtung (105) auszutauschen, die sich nicht auf der rotierenden Komponente befindet.

4. Zentrifugenanordnung nach Anspruch 3, wobei die elektronische Vorrichtung (103) dazu eingerichtet ist, Daten drahtlos über eines oder mehrere von Bluetooth, Wi-Fi und Nahfeldkommunikation auszutauschen.

5. Zentrifugenanordnung nach einem der Ansprüche 3 und 4, wobei die aktive elektronische Vorrichtung (103) eine erste drahtlose Datenaustauschvorrichtung (107) umfasst und die andere Vorrichtung (105), die sich nicht auf der rotierenden Komponente befindet, eine zweite drahtlose Datenaustauschvorrichtung (109) umfasst, wobei die erste und die zweite drahtlose Datenaustauschvorrichtung (107, 109) dazu eingerichtet sind, miteinander Daten auszutauschen.

6. Zentrifugenanordnung nach einem der Ansprüche 2 bis 5, wobei die andere Vorrichtung (107), die sich nicht auf der rotierenden Komponente befindet, eine Steuerung umfasst.

7. Zentrifugenanordnung nach einem der Ansprüche 1 bis 6, wobei die aktive elektronische Vorrichtung (103) einen Sensor (103a) umfasst.

8. Zentrifugenanordnung nach Anspruch 7, wobei der Sensor (103a) dazu eingerichtet ist, ein oder mehrere Charakteristika von Blut und/oder Blutkomponenten zu erfassen.

9. Zentrifugenanordnung nach Anspruch 8, wobei der Sensor (103a) einen optischen Sensor umfasst.

10. Zentrifugenanordnung nach Anspruch 7, wobei der Sensor (103a) dazu eingerichtet ist, ein oder mehrere Charakteristika der Zentrifugenanordnung zu erfassen.

11. Zentrifugenanordnung nach einem der Ansprüche 1 bis 6, wobei die aktive elektronische Vorrichtung (103) eines oder mehrere von einem Blutkomponentendetektor, einem Schnittstellendetektor, einem Drucksensor, einem Temperatursensor, einem Hämolysesensor, einer Waage und einer Kamera umfasst.

12. Zentrifugenanordnung nach Anspruch 1, wobei die rotierende Komponente ferner eine rotierende Kammeranordnung (52) umfasst, die eine Verarbeitungskammer (22) trägt, wobei ein Fluidkreislauf (24) dazu eingerichtet ist, Fluide zu und von der Verarbeitungskammer (22) zu fördern.

13. Nichtchirurgisches und nichttherapeutisches Verfahren zur Verarbeitung von Blut und/oder Komponenten mit einer Zentrifugenanordnung nach Anspruch 1, umfassend:
Zuführen von Blut und/oder Blutkomponenten zu der rotierenden Komponente der Zentrifugenanordnung;
Rotieren der rotierenden Komponente der Zentrifugenanordnung relativ zu der stationären Plattform; und
Versorgen einer aktiven elektronischen Vorrichtung, die der rotierenden Komponente zugeordnet ist, mit Strom mittels einer kontaktlosen Stromquelle.

14. Nichtchirurgisches und nichttherapeutisches Verfahren nach Anspruch 13, ferner umfassend den Austausch von Daten zwischen der aktiven elektronischen Vorrichtung und einer anderen Vorrichtung, die sich nicht auf der rotierenden Komponente befindet.

15. Nichtchirurgisches und nichttherapeutisches Verfahren nach Anspruch 14, wobei der Austausch von Daten zwischen der aktiven elektronischen Vorrichtung und der anderen Vorrichtung das drahtlose Austauschen von Daten umfasst.

## Revendications

1. Ensemble de centrifugeuse destiné au traitement de sang et de composants sanguins, comprenant :
une plateforme stationnaire (48) ;
un composant rotatif situé au-dessus de la plateforme stationnaire (48) et pouvant tourner par rapport à la plateforme stationnaire (48), le composant rotatif comprenant un ensemble d'étrier (50) ayant une base d'étrier (58) ;
un arbre d'entraînement (54) fixé à une base d'étrier (58) ;
un moteur (66) maintenu par la plateforme stationnaire (48), le moteur (66) faisant tourner l'arbre d'entraînement (54) par rapport à la plateforme stationnaire (48), faisant ainsi tourner l'ensemble d'étrier (50) par rapport à la plateforme stationnaire (48) ;
une source d'alimentation sans contact (120) comprenant un transmetteur de source d'alimentation (122) et un récepteur de source d'alimentation (124), dans laquelle le transmetteur de source d'alimentation (122) est configuré pour transmettre sans fil de l'alimentation au récepteur de source d'alimentation (124), le transmetteur de source d'alimentation (122) étant situé au-dessous de l'ensemble d'étrier (50) et associé à une surface (126) de la plateforme stationnaire (48) faisant face à l'ensemble d'étrier (50), et le récepteur de source d'alimentation (124) étant situé au-dessus de la plateforme stationnaire (48) et associé à une surface (128) de l'ensemble d'étrier (50) faisant face à la plateforme stationnaire (48) ; et
un dispositif électronique actif (103) associé au composant rotatif et étant situé au-dessus de la source d'alimentation sans contact (120), le dispositif électronique actif (130) étant relié de manière opérationnelle au récepteur de source d'alimentation (124), dans laquelle le récepteur de source d'alimentation (124) est configuré pour fournir de l'alimentation au dispositif électronique actif (130).

2. Ensemble de centrifugeuse selon la revendication 1, dans lequel le dispositif électronique actif (103) est configuré pour échanger des données avec un autre dispositif qui n'est pas situé sur le composant rotatif.

3. Ensemble de centrifugeuse selon la revendication 2, dans lequel le dispositif électronique actif (103) est configuré pour échanger sans fil des données avec l'autre dispositif (105) qui n'est pas situé sur le composant rotatif.

4. Ensemble de centrifugeuse selon la revendication 3, dans lequel le dispositif électronique (103) est configuré pour échanger sans fil des données via un ou plusieurs parmi Bluetooth, Wi-Fi et la communication en champ proche.

5. Ensemble de centrifugeuse selon l'une quelconque des revendications 3 et 4, dans lequel le dispositif électronique actif (103) comprend un premier dispositif d'échange de données sans fil (107) et l'autre dispositif (105) qui n'est pas situé sur le composant rotatif comprend un second dispositif d'échange de données sans fil (109), dans lequel les premier et second dispositifs d'échange de données sans fil (107, 109) sont configurés pour échanger des données l'un avec l'autre.

6. Ensemble de centrifugeuse selon l'une quelconque des revendications 2 à 5, dans lequel l'autre dispositif (107) qui n'est pas situé sur le composant rotatif comprend un contrôleur.

7. Ensemble de centrifugeuse selon l'une quelconque des revendications 1 à 6, dans lequel le dispositif électronique actif (103) comprend un capteur (103a).

8. Ensemble de centrifugeuse selon la revendication 7, dans lequel le capteur (103a) est configuré pour détecter une ou plusieurs caractéristiques de sang et/ou de composants sanguins.

9. Ensemble de centrifugeuse selon la revendication 8, dans lequel le capteur (103a) comprend un capteur optique.

10. Ensemble de centrifugeuse selon la revendication 7, dans lequel le capteur (103a) est configuré pour détecter une ou plusieurs caractéristiques de l'ensemble de centrifugeuse.

11. Ensemble de centrifugeuse selon l'une quelconque des revendications 1 à 6, dans lequel le dispositif électronique actif (103) comprend un ou plusieurs parmi un détecteur de composant sanguin, un détecteur d'interface, un capteur de pression, un capteur de température, un capteur d'hémolyse, une balance et une caméra.

12. Ensemble de centrifugeuse selon la revendication 1, dans lequel le composant rotatif comprend en outre un ensemble de chambre rotative (52) portant une chambre de traitement (22), dans lequel un circuit fluidique (24) est configuré pour acheminer des fluides vers et depuis la chambre de traitement (22).

13. Procédé non chirurgical et non thérapeutique de traitement de sang et/ou de composants à l'aide d'un ensemble de centrifugeuse selon la revendication 1, comprenant :
l'écoulement de sang et/ou de composants sanguins vers le composant rotatif de l'ensemble de centrifugeuse ;
la rotation du composant rotatif de l'ensemble de centrifugeuse par rapport à la plateforme stationnaire ; et
l'alimentation d'un dispositif électronique actif associé au composant rotatif au moyen d'une source d'alimentation sans contact.

14. Procédé non chirurgical et non thérapeutique selon la revendication 13, comprenant en outre l'échange de données entre le dispositif électronique actif et un autre dispositif qui n'est pas situé sur le composant rotatif.

15. Procédé non chirurgical et non thérapeutique selon la revendication 14, dans lequel l'échange de données entre le dispositif électronique actif et l'autre dispositif comprend l'échange sans fil de données.
